# EUROPEAN PATENT APPLICATION

(11) **EP 2 671 534 A1**
(43) Date of publication of application: **11.12.2013**
(21) Application number: 12004304.7
(22) Date of filing: 06.06.2012
(51) Int. Cl.: A61C 5/06, B65D 83/00, A61J 1/20

(54) **Cartridge and dispenser**

(71) Applicant: Dentsply DeTrey GmbH, 78467 Konstanz (DE)
(72) Inventor: Evers, Markus, 78476 Allensbach (DE)
(74) Representative: Hartz, Nikolai

(57) **Abstract**

Cartridge (10) for storing and dispensing a flowable composition, which comprises
(a) a hollow elongated body (11) having a proximal end (110) and a distal end (111); said elongated body (11) forming a storage chamber (112) having a first opening (113) at the proximal end (110) and a second opening (114) at the distal end (111);
(b) a piston (12) slidably inserted into the first opening (113) of the elongated body (11) for sealingly closing the storage chamber (112);
(c) a discharge nipple (13) having an external thread (131), which is provided at the second opening (114), whereby the external thread has a handedness defining a first direction of rotation in which an internal thread (311) engaging the external thread (131) of the discharge nipple (13) moves away from the elongated body (11); wherein the elongated body (11) is provided with a sleeve (115) at the proximal end (110), said sleeve (115) having a rack of one or more asymmetrical teeth (116) provided on the circumference of the sleeve (115), with each tooth (116) having a steep slope (117) facing the first direction and a moderate slope (118) facing a second direction opposite to the first direction.

## Description

### Field of the Invention

The present invention relates to a cartridge for storing and dispensing a flowable composition. The flowable composition may be selected from a dental composition, a medical composition, a pharmaceutical composition, or a cosmetic composition. The cartridge may contain a single component or multiple components.

Moreover, the present invention relates to a dispenser for receiving and holding the cartridge of the present invention when the flowable composition is dispensed. A cartridge of the present invention may be adapted for mixing multiple components. Moreover, the present invention relates to a system comprising the cartridge and the dispenser of the present invention, and a process for using the system.

Finally, the present invention relates to the use of the cartridge of the present invention for storing and dispensing a flowable composition whereby the flowable composition may be a single component flowable composition or a multicomponent flowable composition.

### Background of the Invention

W02009053851 discloses a dispenser comprising: a body, at least a portion of the body being substantially cylindrical; a tank constructed and configured to contain a flowable material; a tip, the tip comprising an opening through which the flowable material is dispensed; an ejection mechanism constructed and configured to urge the flowable material from the tank through the opening in the tip; and an advancing mechanism constructed and configured to move the ejection mechanism within the tank; wherein the body defines a proximal end of the dispenser, and the tip defines a distal end of the dispenser, at least the proximal end comprises a cross-section which differs from the cross-section of the remainder of the body and is configured to be easily grasped by a human hand.

Moreover, a pen-shaped delivery form is known as VivaPen (IvoclarVivadent) which enables direct application of a dental adhesive in the mouth of the patient. The device may be operated with the click mechanism on the side of the pen. If additional adhesive is required, a click is required to dispense more material. Due to the exchangeable brush cannulas, the device can be used for up to 100 applications. However, once the supply of the device has been exhausted, a new device is required. It is not possible to refill the device or to exchange a cartridge in order to continue using the device. Moreover, the exchangeable brush cannulas are snap-on cannula brush tips which do not allow for a free rotational adjustment of the orientation of the bent cannulas around the longitudinal axis of the device. Although a 270 ° rotation is possible, any further rotation will disconnect the cannula from the device.

W02007126532 discloses a single-dose dual fluid cartridge for use with hand-held applicators.

### Summary of the Invention

It is the problem of the present invention to provide a cartridge which is suitable for use in a pen-like dispenser and which allows storage and dispensing of a flowable composition for multiple applications while at the same time allowing a stable and adjustable mounting of an applicator tip and easy cleaning of the discharge nipple between the application of the flowable composition.

Moreover, it is the problem of the present invention to provide a dispenser for the cartridge of the present invention.

The present invention provides a cartridge for storing and dispensing a flowable composition, which comprises
(a) a hollow elongated body having a proximal end and a distal end; said elongated body forming a storage chamber having a first opening at the proximal end and a second opening at the distal end;
(b) a piston slidably inserted into the first opening of the elongated body for sealingly closing the storage chamber;
(c) a discharge nipple having an external thread, which is provided at the second opening, whereby the external thread has a handedness defining a first direction of rotation in which an internal thread engaging the external thread of the discharge nipple moves away from the elongated body;
wherein the elongated body is provided with a sleeve at the proximal end, said sleeve having a rack of one or more asymmetrical teeth provided on the circumference of the sleeve, with each tooth having a steep slope on an edge facing the first direction and a moderate slope facing a second direction opposite to the first direction.

Moreover, the present invention also provides a dispenser comprising
(i) a housing for receiving and holding the cartridge of the present invention so that the discharge nipple projects outside of the housing;
(ii) an actuator assembly comprising a plunger for actuating the piston of the cartridge;
whereby the dispenser further comprises a stationary member engaging the sleeve of the cartridge so that rotary motion of the cartridge is prevented in the first direction of rotation when the stationary member engages the steep slope of a tooth of the sleeve a while rotation of the sleeve member is possible in the second direction of rotation opposite to the first direction.

The present invention also provides a system comprising a cartridge and a dispenser.

The present invention also provides a process for using the system of the invention, which comprises
(A) introducing the cartridge in to the dispenser;
(B) attaching the applicator tip to the cartridge by screwing the internal thread onto the external thread of the discharge nipple of the dispenser;
(C) adjusting the orientation of the needle portion by rotating the cartridge in the second direction.

Finally, the present invention provides a use of the cartridge of the present invention for storing and dispensing a flowable composition.

The cartridge and the dispenser of the present invention are adapted for multiple applications of a flowable material. According to the present invention, an applicator tip may be mounted on the discharge nipple of the cartridge when the cartridge is received and held in the dispenser. The discharge nipple is provided with an external thread engaging the internal thread of the applicator tip. The external thread defines a first direction of rotation in which an internal thread engaging the external thread of the discharge nipple moves away from the cartridge. A second direction of rotation may be defined as the direction opposite to the first direction. Accordingly, the applicator tip may be mounted on the cartridge by rotating the application tip engaging the discharge nipple in the second direction. When the applicator tip fully engages the discharge nipple, a torque limit is encountered. When a torque is applied which exceeds the torque limit, the cartridge and the applicator tip may be rotated while applicator tip and the cartridge remain essentially in the same orientation relative to each other. Accordingly, the orientation of the cartridge applicator unit relative to the dispenser may be adjusted by free rotation in the second direction. On the other hand, when the applicator tip is rotated in the first direction, the cartridge will only follow until an unsymmetrical tooth of a rack of teeth engages with a static member in the dispenser so that rotation of the cartridge in the first direction is prevented. Accordingly, a second torque limit is encountered. When further torque is applied, the applicator tip may be rotated independently from the cartridge in the first direction whereby the internal thread of the applicator tip moves away from the cartridge so that the applicator tip may eventually be unmounted.

According to the present invention, the use of an external thread on the discharge nipple provides a surface which may be easily cleaned and disinfected after use contrary to a conventional luer lock.

### Brief Description of the Figures

Figure 1 is a perspective side view of a preferred embodiment of the cartridge according to the present invention.

Figure 2 is a cross-sectional side view of the preferred embodiment of the cartridge according to the present invention shown in figure 1.

Figure 3 is a perspective side view of a piston for use in a cartridge of the present invention.

Figure 4 is a perspective side view of the piston for use in a cartridge of the present invention shown in figure 3 whereby the sealing members have been removed.

Figure 5 is a side view of a preferred embodiment of an applicator tip for a cartridge according to the present invention.

Figure 6 is cross-sectional side view of the preferred embodiment of the applicator tip for a cartridge according to the present invention shown in figure 5.

Figure 7 is a perspective front view of a preferred embodiment of a dispenser according to the present invention, which holds a cartridge of the present invention.

Figure 8 is a cross-sectional side view of the preferred embodiment of a dispenser shown in figure 7, which holds a cartridge of the present invention.

Figure 9 is a perspective front view of a preferred embodiment of a dispenser according to the present invention, without cartridge.

Figure 10 is a cross-sectional side view of the preferred embodiment of a dispenser shown in figure 9, without cartridge.

Figure 11 is an exploded view drawing of a preferred embodiment of a dispenser according to the present invention, without cartridge.

Figure 12 is a side view of a proximal end of a cartridge of the present invention, including the sleeve portion having a rack of unsymmetrical teeth cooperating with a stationary member of the dispenser having engaged the sleeve a moderate slope of an unsymmetrical tooth.

Figure 13 is a side view of a proximal end of a cartridge of the present invention, including the sleeve portion having a rack of unsymmetrical teeth cooperating with a stationary member of the dispenser having engaged the sleeve a steep slope of an unsymmetrical tooth.

### Description of the Preferred Embodiments

The present invention provides a cartridge for storing and dispensing a flowable composition. The flowable composition is preferably a flowable material having a low viscosity. In particular, the flowable composition has a dynamic viscosity at 23 °C of less than 1500 Pas, more preferably less than 500 Pas.

The cartridge according to the present invention may preferably hold a composition selected from a dental composition, a medical composition, a pharmaceutical composition, or a cosmetic composition. Examples of preferred dental materials are dental bonding agents including dental adhesives, flowable dental composites, dental bleaching agents, and dental pit and fissure sealants. The flowable composition may also be a medical composition such as a medical adhesive or medical bone cement, or a pharmaceutical composition such as an insulin formulation, or a cosmetic composition such as skin cosmetic composition. The flowable composition may a one-component flowable composition or a multi-component flowable composition, whereby the multiple flowable components of the multi-component flowable composition are stored in separate compartments in the cartridge and mixed when the multicomponent flowable composition is dispensed.

Preferably, the flowable composition is a flowable dental composition having a dynamic viscosity at 23 °C of less than 1500 Pas and is selected from a dental bonding agent, a pit and fissure sealant, an etching gel, a flowable dental composite, a root canal sealer, and a rinsing solution.

The cartridge comprises
(a) a hollow elongated body having a proximal end and a distal end; said elongated body forming a storage chamber having a first opening at the proximal end and a second opening at the distal end;
(b) a piston slidably inserted into the first opening of the elongated body for sealingly closing the storage chamber;
(c) a discharge nipple having an external thread, which is provided at the second opening, whereby the external thread has a handedness defining a first direction of rotation in which an internal thread engaging the external thread of the discharge nipple moves away from the elongated body;
wherein the elongated body is provided with a sleeve at the proximal end, said sleeve having a rack of one or more asymmetrical teeth provided on the circumference of the sleeve, with each tooth having a steep slope on an edge facing the first direction and a moderate slope facing a second direction opposite to the first direction.

The cartridge may further comprise (e) a cap having an internal thread for mating with the external thread of the discharge nipple. The cap protects the discharge nipple during storage and shields the flowable composition from the environment. The cap may be color coded or display printed information, for example for identifying the content of the cartridge.

The cartridge may further comprise a film provided at the proximal end for sealing the first opening.

According to a preferred embodiment, the cartridge according to the present invention has an essentially axisymmetric outer contour so that the cartridge may rotate in the dispenser in the first direction.

The present invention also provides a dispenser. The dispenser can take the general form of a pen-like device which is sized and configured to be easily grasped by the human hand. The dispenser comprises a housing for receiving and holding the cartridge of the present invention so that the discharge nipple projects outside of the housing.

The dispenser further comprises an actuator assembly comprising a plunger for actuating the piston of the cartridge.

The dispenser further comprises a stationary member engaging the sleeve of the cartridge so that rotary motion of the cartridge is prevented in the first direction of rotation when the stationary member engages the steep slope of a tooth of the sleeve a while rotation of the sleeve member is possible in the second direction of rotation opposite to the first direction.

According to the present invention, the dispenser may have a plunger comprising a rack for engaging the cartridge at the first opening and sliding the piston in the storage compartment toward the second opening.

The actuator assembly may further comprise an actuator member pivotably supported by the housing and engaging the toothed rack of the plunger.

The present invention also provides a system comprising a cartridge of the present invention and a dispenser of the present invention.

Preferably, the system may further comprise an applicator tip comprising
(1) a proximal portion including an internal thread mating with the external thread of the discharge nipple of the cartridge and
(2) a bent needle portion having an outlet orifice which is offset from the longitudinal axis of the cartridge.

According to a preferred embodiment, the needle portion comprises a fiber flocking proximate to the outlet orifice for retaining a volume of flowable composition.

The present invention further provides a process for using the system, which comprises
(A) introducing the cartridge in to the dispenser;
(B) attaching the applicator tip to the cartridge by screwing the internal thread onto the external thread of the discharge nipple of the dispenser;
(C) adjusting the orientation of the needle portion by rotating the cartridge in the second direction.

Finally, the present invention provides the use of a cartridge of the present invention for storing and dispensing a flowable composition.

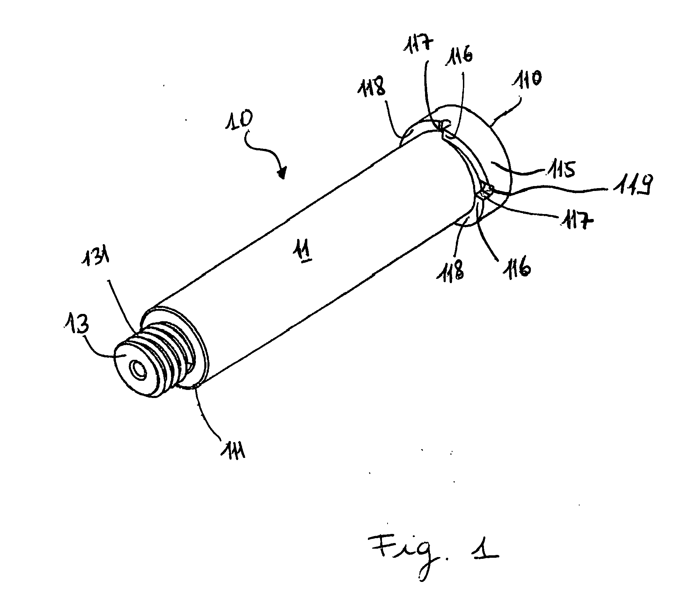

Figure 1 shows a perspective side view of a cartridge 10 for storing and dispensing a flowable composition according to a preferred embodiment of the present invention. The cartridge comprises a hollow elongated body 11. The hollow elongated body 11 may be made of any material suitable for storing and dispending a flowable composition. Preferably, the hollow elongated body 11 is made of a resin material. A preferred example of a resin material is selected from polyethylene, polypropylene or a polycycloalkylene resin. In view of the delicate nature of the flowable compositions stored in the cartridge, the selection of the material of the cartridge 10 is relevant for fulfilling the requirements such as with regard to the shielding of the flowable composition against irradiation, the sealing property of the material in view of the low viscosity, solvents and monomers in the flowable composition, and the stabilization of the flowable composition by oxygen transferred from the environment into the flowable composition. The selection of the materials is also important with regard to the requirement for the application of minuscule amounts of flowable composition.

The hollow elongated body 11 has a proximal end 110 and a distal end 111. The proximal end is associated with a plunger rod actuating a piston 12. The distal end 111 is associated with the dispensing of the flowable composition through an applicator tip. Accordingly, the elongated body 11 forms a storage chamber 112 having an opening 113 at the proximal end 110 and a second opening 114 at the distal end 111. The volume of the storage chamber is not particularly limited as long as a pen-like device having favourable handling properties may be provided. According to a preferred embodiment, the storage chamber has a volume of 0.01 to 10 ml, more preferably 0.5 to 2 ml.

The storage chamber has at least two openings. The first opening 113 is at the proximal end and, preferably, centred on the longitudinal axis of the hollow elongated body 11. The first opening 113 serves for the insertion of a piston 12 which is slidably deplacable along the longitudinal axis of the elongated body 11 in the storage chamber 112. The piston 12 sealingly closes the storage chamber 112 and prevents any flowable composition from being urged backwards through the first opening 113 at the proximal end 110 of the storage chamber 112.

In case of a two-component flowable composition, the piston may comprise an additional storage chamber which is separate from the storage chamber 112 for storing and dispensing an additional flowable material. The piston then also comprises a static delivery tube which cooperates as an additional piston with the piston for dispensing the additional flowable material as disclosed in W02007126532.

The cartridge 10 further comprises a discharge nipple 13 having an external thread 131. The discharge nipple 13 is provided at the second opening 114 and preferably forms an integral part of the hollow elongated body 11. The external thread 131 of the discharge nipple has a handedness. The handedness defines a first direction of rotation in which an internal thread engaging the external thread 131 of the discharge nipple moves away from the elongated body 11. In general, it is not of particular importance which handedness is chosen. The external thread 131 provided on the discharge nipple 13 may be left-handed or right-handed. However, in view of the intuitive handling of the cartridge 10, it is preferable to provide a right-handed external thread 131.

The elongated body 11 is provided with a sleeve 115 at the proximal end. Preferably, the sleeve 115 forms an integral part of the hollow elongated body 11. The sleeve has rotational symmetry based on a rack of one or more asymmetrical teeth 116 provided on the circumference of the sleeve 115.

In Figure 1, the sleeve 115 has four-fold rotational symmetry whereby four equally spaced asymmetrical teeth are provided. Each asymmetrical tooth has a steep slope 117 and a moderate slope 118. The steep slope 117 and the moderate slope 118 are contiguous through a portion functioning as a locking recess 119. In an alternative embodiment, a locking recess 119 may be omitted. The number of equally spaced asymmetrical teeth 116 is not particularly limited. However, for reasons of stability at least two equally spaced asymmetrical teeth 116 should be provided.

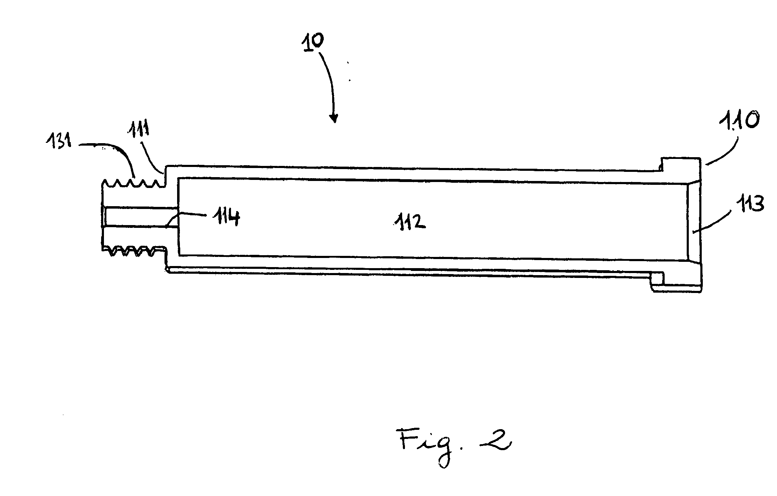

As shown by Figure 2, the cross-sectional view of the cartridge 10 shows uniform wall thicknesses of the hollow elongated body 11 along the storage chamber 112. However, the proximal portion comprising the sleeve 115 may require an increased wall thickness. Likewise, the distal portion including the discharge nipple 13 has an increased wall thickness to accommodate the external thread 131. The length of the hollow elongated body 11 is typically in the range of from 3 to 12 cm. The diameter of the hollow elongated body 11 is usually in the range of from 4 mm to 2 cm.

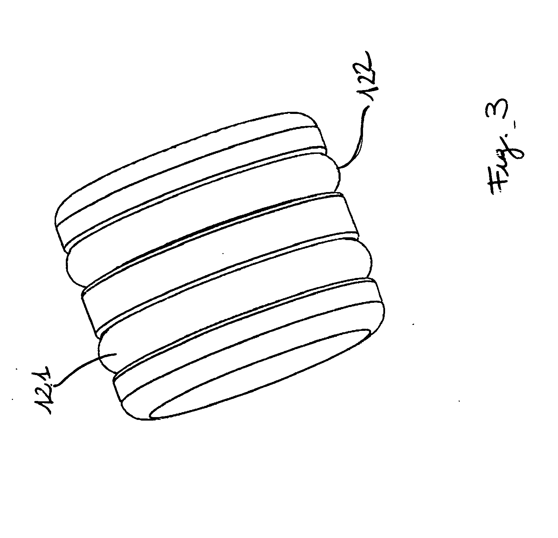

Figure 3 shows an embodiment of a piston 12 for use with the cartridge 10 of the present invention. Accordingly, the piston 12 is provided with sealing rings 121, 122 for providing a tight seal of the storage chamber 112. The sealing rings 121, 122 are preferably made of an elastic material such as an elastomer. Alternatively, the sealing rings may be annular sealing protrusions integrally formed with the piston side walls, i.e. during moulding of the piston.

The piston 12 preferably is made of a rigid material so that the actuation of the plunger rod may be directly translated into a sliding movement of the piston 12, thereby providing a desired amount of dispensed flowable composition with high accuracy. The distal face 124 of the piston may be flat. Alternatively, the distal face may have a concave shape so that the force applied during extrusion of the flowable material enhance the sealing effect of annular sealing protrusions provided on a flexible portion of the distal end of the piston.

The material of the piston 12 may be the same or different from the material of the hollow elongated body and the discharge nipple 13.

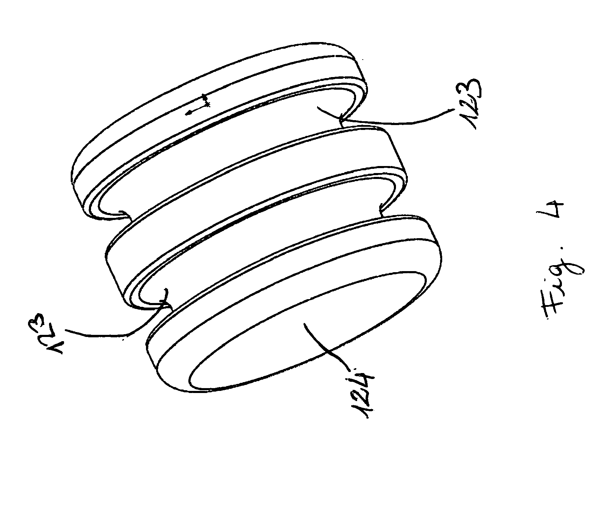

As shown by Figure 4, the sealing rings 121, 122, are received in sealing ring recesses 123 at the circumference of the piston. The depth of the sealing ring recesses 123 must be adapted so that the sealing rings 121, 122 are securely seated during the sliding motion of the piston 12 in the hollow elongated member 11 of the cartridge 10 during actuation.

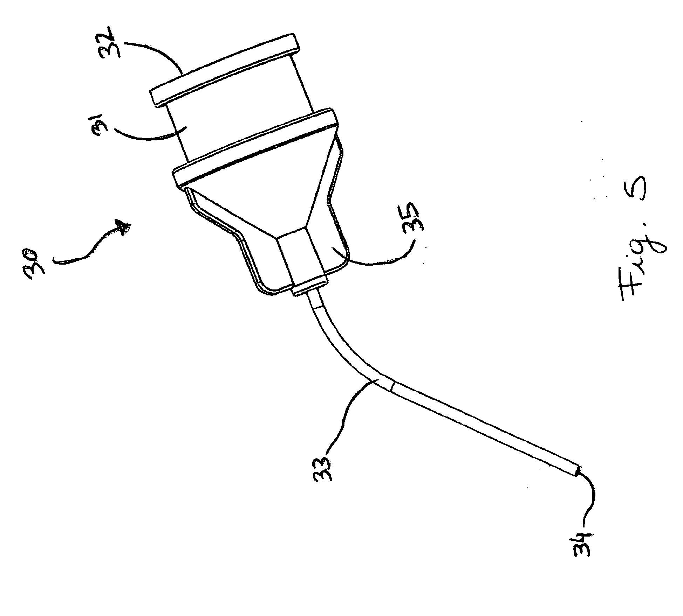

Figure 5 shows an embodiment of an applicator tip 30 of a preferred embodiment of the present invention. The applicator tip 30 comprises a proximal portion 31. The proximal portion includes an internal thread 311 mating with the external thread 131 of the discharge nipple 13 of the cartridge 10. The applicator tip 30 further comprises a bent needle portion 33 having an outlet orifice 34 which is offset from the longitudinal axis of the cartridge 10. The applicator tip 30 may further comprise multiple ribs 35 for stabilizing the connection of the bent needle portion 33 with the proximal portion 31. Moreover, the ribs serve as a gripping aid when the applicator tip is mounted on the external thread of the cartridge. The ribs are useful for providing a torque which is sufficient to fasten and disconnect the applicator tip from the cartridge. Moreover, the ribs 35 facilitate the torque for orienting the assembly of the applicator tip and the cartridge so as to direct the bent needle portion 33 in the desired angular position.

In case of a multi-component flowable composition, the applicator tip may be provided with a static mixer within it. The static mixer mixes the flowable materials stored in the cartridge as they are dispensed from the cartridge.

The applicator tip 30 is a disposable item. In order to avoid cross-contamination or even infection of a patient, the applicator tip 30 must be disposed of after each treatment. Given that the dispenser 20 and the cartridge 10 of the present invention are useful for multiple uses, it is important that the discharge nipple 13 of the cartridge 10 may be efficiently cleaned and disinfected. Accordingly, an external thread 131 of the discharge nipple of the cartridge 10 of the present invention is superior with regard to the cleaning and disinfecting as compared to an internal thread. Since the internal thread is provided on the disposal applicator tip, a hygiene problem does not arise with the applicator tip of the present invention.

The bent needle portion 33 is preferably made of metal. The proximal portion of the applicator tip 31 is preferably made of a polymeric material. The connection between the metal bent needle portion 33 and the polymeric proximal portion 31 of the applicator tip 30 is preferably provided by an adhesive.

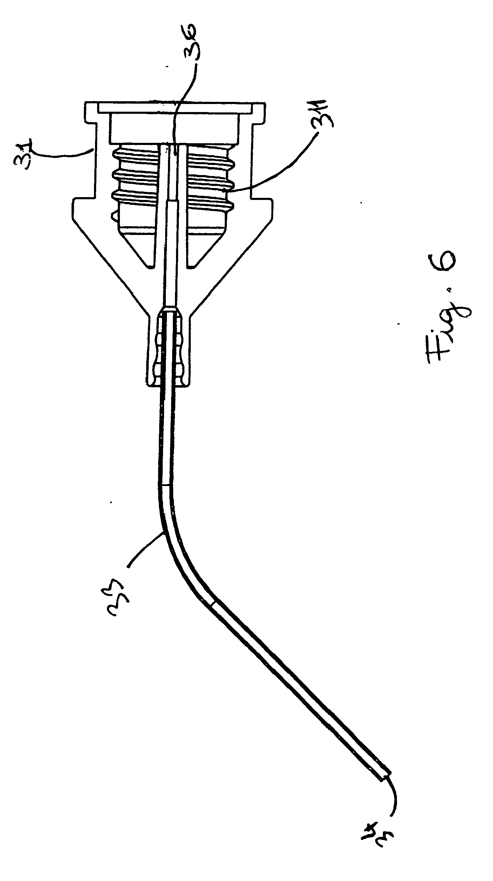

Figure 6 shows a cross-sectional view of the applicator tip 30 of the present invention. Accordingly, within the applicator tip 30, a protrusion 36 is provided which is introduced into the outlet orifice 114 of the discharge nipple 13 of the cartridge 10 when the applicator tip 30 is mounted on the cartridge 10. Accordingly, a clean transition between the outlet orifice and the applicator tip 30 may be provided which is essential for avoiding a permanent connection between the applicator tip 30 and the cartridge 10 due to the adhesive properties of the flowable composition. The diameter of the through bore of the bent needle portion 33 may be adjusted to the volume desired to be dispensed from the cartridge. Typically, the diameter of the through bore is in the range of 0.1 to 1 mm.

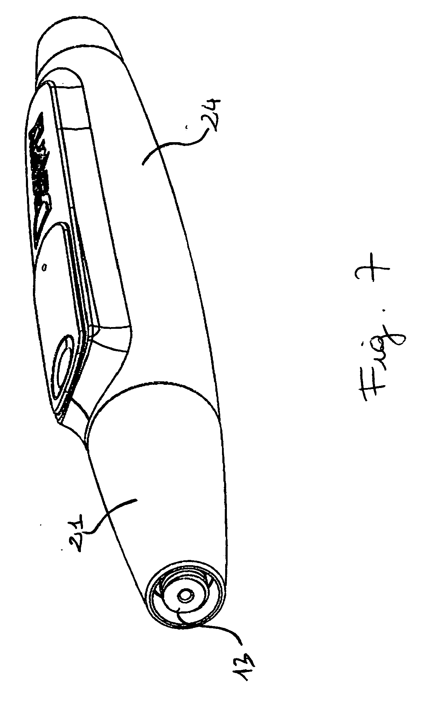

Figure 7 shows a perspective side view of a dispenser 20 of the present invention which holds a cartridge 10 of the present invention. The dispenser 20 comprises a housing 21 for receiving and holding the cartridge 10 of the present invention and a body portion 24 containing an actuator assembly 22 comprising a plunger 221 for actuating the piston 12 of the cartridge 10.

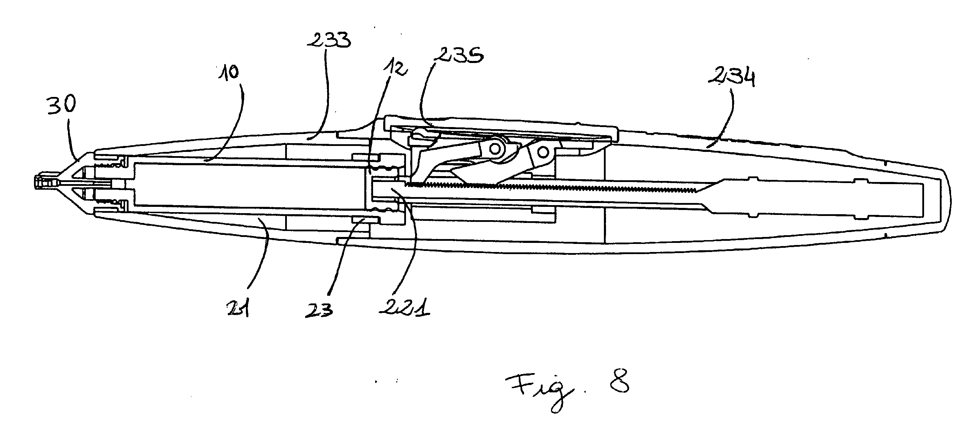

In Figure 8, a cross-sectional view of the dispenser of the invention is shown. Accordingly, the distal portion 233 of the dispenser may be detached from the proximal portion 234 of the dispenser. The distal portion 233 includes a housing 21 for receiving and holding the cartridge 10 of the invention. The proximal portion includes an actuator assembly 22 comprising a plunger 221 for actuating the piston 12 of the cartridge 10. The distal portion 233 and the proximal portion 234 may be combined by a snap-fit connection. Alternatively, it is possible to combine the distal portion and the proximal portion with any other connection which provides a reversible attachment for the housing 21 so that the cartridge may be inserted into the stationary member 23 and pushed forward towards an opening so that the external thread 131 of the discharge nipple 13 is displayed for attachment of the applicator tip 30. In Figure 8, the applicator tip 30 is attached to the cartridge 10 whereby the cartridge 10 is securely fastened between the stationary element 23 and the abutment at the inside of the housing in the distal portion 233 of the dispenser 20. The proximal portion 234 of the dispenser 20 is adapted to project a plunger 221 into a recess provided in the piston 12 of the cartridge 10 and to exert pressure on to the piston 12 when the actuating assembly 22 moves the piston 12 further inside the cartridge 10. For actuating, the practitioner pushes on to the actuating assembly 22 in a direction perpendicular to the longitudinal axis of the dispenser 20.

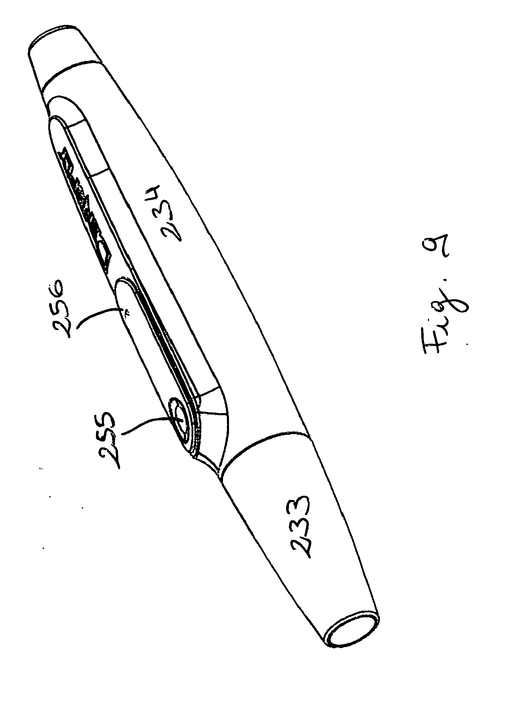

Figure 9 shows a perspective view of the dispenser 20 of the present invention without the cartridge 10. Accordingly, the distal portion 233 does not display the discharge nipple 13 of a cartridge. As shown, the proximal portion 234 includes an elongated body extending along a substantial part of the proximal portion of the dispenser 20 wherein an actuator assembly 22 is accommodated. The elongated body also includes a dispensing actuator 255 used for actuating the plunger. The elongated body further includes a release actuator 256 used for releasing the plunger so that the plunger 221 may be returned to a retracted position. The dispenser 20 also includes a release mechanism so that the plunger may be retracted after exchange of the cartridge.

Accordingly, once a cartridge is inserted into the dispenser, the plunger may be moved forward by actuating the actuator button whereby every actuation will provide a defined amount of flowable composition. Once the plunger has been moved to its forward most position, the cartridge is empty. Accordingly, it is necessary to retract the plunger to its original position so that a new cartridge may be accommodated in the dispenser. For this purpose, a retraction button is pushed and the plunger is manually brought into its original position.

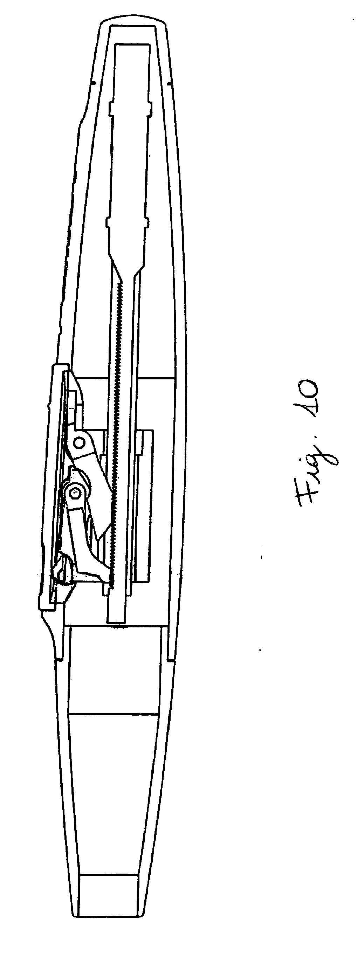

Figure 10 shows a cross-sectional view of the dispenser without cartridge whereby the plunger is in its initial retracted position before a cartridge is inserted.

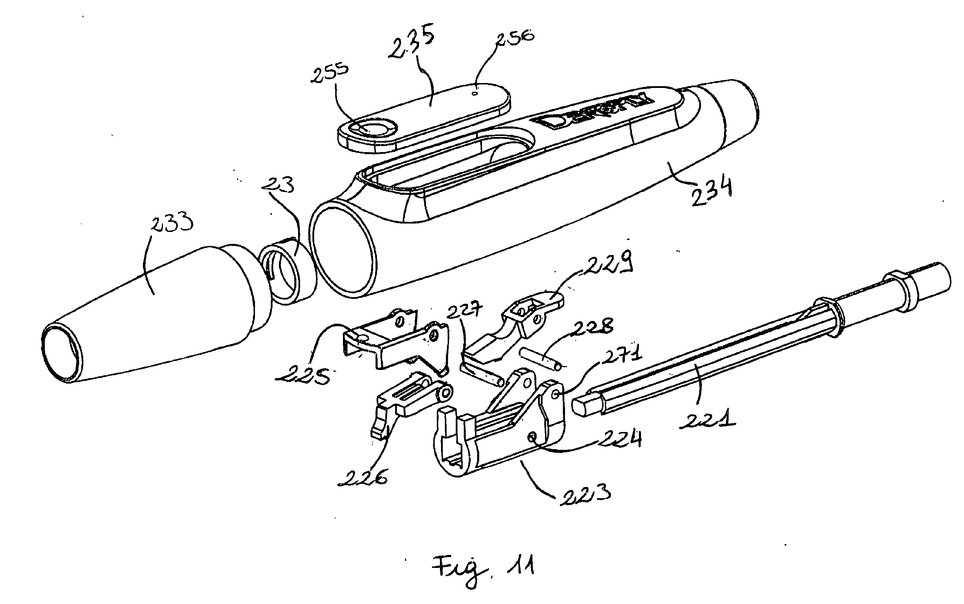

Figure 11 shows an exploded side view of the dispenser 20 of the present invention. In particular, the distal portion 233 of the dispenser and the stationary element 23 are shown as separate parts which may be combined with the proximal portion 234 of the dispenser 20. As shown, the elongated body of the proximal portion 234 of the dispenser 20 includes a lid 235 which covers the actuating assembly 22.

The actuator assembly 22 comprises the plunger 221 which is held and guided by a support element 223 through which the plunger 221 may slide upon actuation. The support element 223 may be locked into place in the proximal portion 234 of the dispenser 20 and exposes an open canal wherein the plunger is seated. The open canal exposes a rack of teeth on the upper surface of the plunger 221 cooperating with actuation means pivotably attached to the support element 223. Specifically, an actuation member 225 is pivotably hinged by a shaft 227 at the position of bore 224. The actuation member 225 receives actuation force from the operator pushing the dispensing actuator 255 on the lid 235 of the dispenser 20 in a direction perpendicular to the longitudinal axis of the dispenser 20. The actuation member 225 transmits the actuation force to a dispensing member 226 pivotably hinged by a shaft 228 at the position of bore 271. The dispensing member 226 engages the rack of teeth of the plunger 221 so that the plunger 221 is moved into the distal direction when the dispensing member 226 pivots about shaft 228 when receiving the force transmitted by the actuation member 225. Upon release of the actuation member, the dispensing member 226 may return to the original position while engaging teeth of the rack of teeth which are located in a more proximal position.

The actuator assembly further comprises a release member 229 hinged by shaft 228 at the position of bore 271. The hinge member 229 may be actuated by exerting a force on the release actuator 256. Accordingly, the release member 229 transmits the force to the dispensing member 226 so that the dispensing member 226 disengages the rack of teeth of the plunger. Accordingly, by pushing on the release actuator 256, the operator may free the plunger from the blocking action of the dispensing member 226 and push the plunger back into a retracted position.

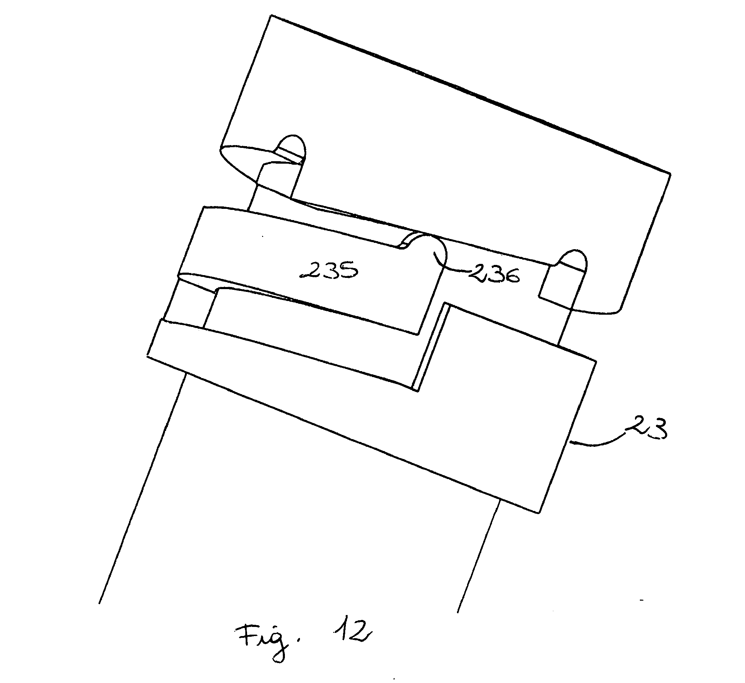

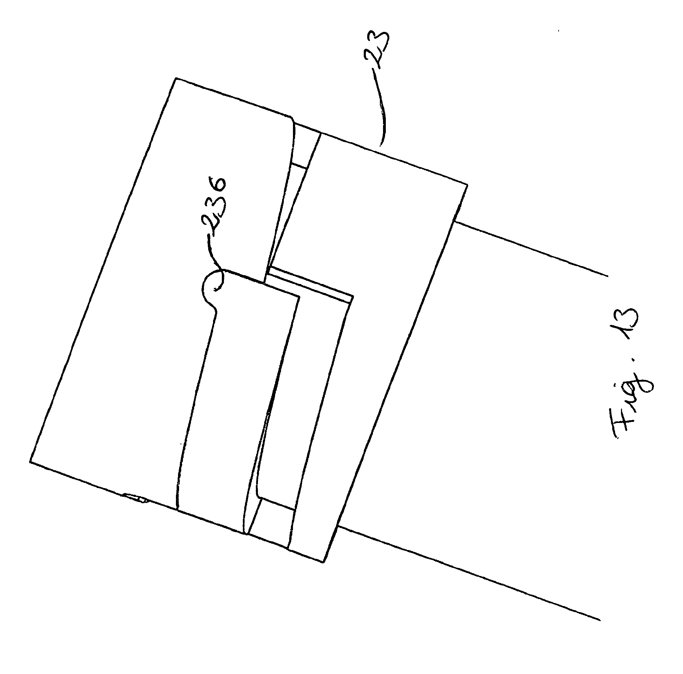

Figure 12 shows the cooperation between the sleeve 115 of a cartridge 10 and the stationary element 23 of the dispenser 20. Accordingly, the stationary element 23 has a resilient arm 235 bending around the external surface of the cartridge 10 and having a protrusion 236 adapted for engaging the locking recess 119 on the sleeve 115 of the cartridge 10. As shown in Figure 12, the protrusion 236 of the stationary member 23 engages a moderate slope 118 of an asymmetrical tooth of a rack of asymmetrical teeth 116 of the sleeve 115 of the cartridge 10. In this position, limited rotation of the cartridge relative to the stationary element 23 is possible until the locking protrusion 236 is securely engaging the locking recess 119 as shown in Figure 13.

Now a process for using the system according to the present invention is explained. In order to introduce a cartridge of the invention into the dispenser, the distal portion is detached from the proximal portion of the dispenser. In the next step, the cartridge is introduced into the dispenser 20 by positioning the discharge nipple 13 through the stationary element 23 and pushing the cartridge 10 through the stationary element 23 to the abutment of the distal portion of the cartridge 10 in the housing of the dispenser. Then, the distal portion of the dispenser is attached to the proximal portion of the dispenser. The cartridge is secured in place by attaching the applicator tip 30 to the cartridge by screwing the internal thread of the applicator tip 30 onto the external thread 131 of the discharge nipple 13 of the dispenser 20.

Subsequently, it is possible to adjust the orientation of the needle portion 33 of the applicator tip 30 by rotating the cartridge 10 in the second direction. For detaching the applicator tip 30, a rotation of the applicator tip in the first direction is required to unscrew the applicator tip from the cartridge 10.

## Claims

1. Cartridge (10) for storing and dispensing a flowable composition, which comprises
(a) a hollow elongated body (11) having a proximal end (110) and a distal end (111); said elongated body (11) forming a storage chamber (112) having a first opening (113) at the proximal end (110) and a second opening (114) at the distal end (111);
(b) a piston (12) slidably inserted into the first opening (113) of the elongated body (11) for sealingly closing the storage chamber (112);
(c) a discharge nipple (13) having an external thread (131), which is provided at the second opening (114), whereby the external thread has a handedness defining a first direction of rotation in which an internal thread (311) engaging the external thread (131) of the discharge nipple (13) moves away from the elongated body (11);
wherein the elongated body (11) is provided with a sleeve (115) at the proximal end (110), said sleeve (115) having a rack of one or more asymmetrical teeth (116) provided on the circumference of the sleeve (115), with each tooth (116) having a steep slope (117) facing the first direction and a moderate slope (118) facing a second direction opposite to the first direction.

2. The cartridge (10) according to claim 1, which further comprises
(e) a cap (14) having an internal thread (141) for mating with the external thread (131) of the discharge nipple (13).

3. The cartridge (10) according to any one of claim 1 or 2, which further comprises a film (15) provided at the proximal end (110) for sealing the first opening (113).

4. The cartridge (10) according to any one of the preceding claims which holds a flowable composition.

5. The cartridge (10) according to claim 4, wherein the flowable composition has a dynamic viscosity at 23 °C of less than 1500 Pas and is selected from a dental bonding agent, a pit and fissure sealant, an etching gel, a flowable dental composite, a root canal sealer, and a rinsing solution.

6. The cartridge (10) according to any one of the preceding claims which is has an essentially axisymmetric outer contour.

7. A dispenser comprising
(i) a housing for receiving and holding the cartridge defined by claim 1 so that the discharge nipple projects outside of the housing;
(ii) an actuator assembly comprising a plunger for actuating the piston of the cartridge;
whereby the dispenser further comprises a stationary member engaging the sleeve of the cartridge so that rotary motion of the cartridge is prevented in the first direction of rotation when the stationary member engages the steep slope of a tooth of the sleeve a while rotation of the sleeve member is possible in the second direction of rotation opposite to the first direction.

8. The dispenser according to claim 7, wherein the plunger comprises a rack for engaging the cartridge at the first opening and sliding the piston in the storage compartment toward the second opening.

9. The dispenser according to claim 8, wherein actuator assembly further comprises an actuator member pivotably supported by the housing and engaging the toothed rack of the plunger.

10. A system comprising a cartridge as defined by any one of claims 1 to 6 and a dispenser as defined by any one of claims 7 to 9.

11. The system according to claim 10, which further comprises an applicator tip comprising
(1) a proximal portion including an internal thread mating with the external thread of the discharge nipple of the cartridge and
(2) a bent needle portion having an outlet orifice which is offset from the longitudinal axis of the cartridge.

12. The system according to claim 11, wherein the needle portion comprises a fiber flocking proximate to the outlet orifice for retaining a volume of flowable composition.

13. A process for using the system as defined by claim 11, which comprises
(A) introducing the cartridge in to the dispenser;
(B) attaching the applicator tip to the cartridge by screwing the internal thread onto the external thread of the discharge nipple of the dispenser;
(C) adjusting the orientation of the needle portion by rotating the cartridge in the second direction.

14. Use of a cartridge as defined by claim 1 for storing and dispensing a flowable composition.
